# FASCICULE DE BREVET EUROPEEN

(11) **EP 3 256 971 B1**
(45) Date de publication et mention de la délivrance du brevet: **24.07.2019**
(21) Numéro de dépôt: 16705234.9
(22) Date de dépôt: 25.01.2016
(51) Int. Cl.: G16H 40/40

(54) **PRESCRIPTION SÉCURISÉE D'UN DISPOSITIF MÉDICAL LOGICIEL**
SICHERES REZEPT EINER MEDIZINISCHEN SOFTWAREVORRICHTUNG
SECURE PRESCRIPTION OF A MEDICAL SOFTWARE DEVICE

(30) Priorité: 12.02.2015 FR 1551148
(43) Date de publication de la demande: 20.12.2017
(73) Titulaire: Voluntis, 75017 Paris (FR)
(72) Inventeur: SIRE, Barthélémy, 92400 Courbevoie (FR); CURRO, Julien, 69008 Lyon (FR); MOALIC, Yannick, 75016 Paris (FR); BUREY, Dominique, 91800 Brunoy (FR); VIAL, Etienne, 78300 Poissy (FR); MARMOT, Romain, Belmont, MA 02478 (US)
(74) Mandataire: Cabinet Plasseraud
(86) Numéro de dépôt international: PCT/FR2016/050147
(87) Numéro de publication internationale: WO 2016/128636

(56) Documents cités:
- US-A1- 2014 188 506
- US-A1- 2014 325 065

## Description

### Domaine technique et Etat de la technique

L'objet de la présente invention a trait au domaine des dispositifs médicaux, et concerne plus particulièrement les dispositifs médicaux dits logiciels.

Un logiciel peut désormais être considéré comme un dispositif médical, ceci pour autant qu'il soit destiné à être utilisé spécifiquement à des fins médicales notamment diagnostiques et/ou thérapeutiques.

On parle de dispositif médical logiciel.

Les fonctionnalités logicielles mises en oeuvre par un dispositif médical logiciel sont bien souvent prévues pour accompagner un patient dans un traitement thérapeutique ; ces fonctionnalités peuvent par exemple assister le patient dans le dosage d'un médicament.

Le logiciel en tant que dispositif médical peut donc influer directement sur la santé du patient.

On comprend ainsi qu'un dispositif médical logiciel favorise la prise en charge médicale du patient, ceci à condition que les fonctionnalités logicielles mises en oeuvre sur le dispositif médical logiciel respectent la prescription médicale établie spécifiquement par le médecin pour son patient.

Inversement, un tel dispositif médical logiciel est susceptible de représenter un risque pour le patient si l'une des fonctionnalités mises en oeuvre par le dispositif médical logiciel n'est pas conforme à la prescription médicale, par exemple si le dosage du médicament n'est pas correct.

Prenons l'exemple concret d'un patient asthmatique : l'utilisation d'un dispositif médical logiciel destiné à accompagner le patient dans le traitement de l'asthme peut permettre la détermination automatique de la dose d'un bronchodilatateur pour éviter ou limiter la crise d'asthme ; ceci peut se faire en fonction notamment :
- d'une information relative au Débit Expiratoire de Pointe (DEP) mesuré à l'aide d'un appareil de mesure adapté, et
- d'une prescription médicale établie par un médecin spécialiste prenant en considération des informations médicales personnelles relatives au patient (par exemple : son type d'asthme, son âge, son poids, son activité régulière, ses éventuels antécédents médicaux, etc.).

Le dosage du bronchodilatateur par le dispositif médical logiciel doit être conforme à la prescription médicale établie par le médecin et à l'état de santé du patient en prenant en considération les informations médicales personnelles ci-dessus.

On comprend ici qu'une erreur dans la détermination de ce dosage peut avoir des conséquences sur la santé du patient, une telle erreur de dosage pouvant dans certains cas extrêmes conduire au décès du patient.

Bien évidemment cet exemple de l'asthme est un exemple parmi d'autres : il est tout à fait possible de prévoir un dispositif médical logiciel pour accompagner le patient dans le traitement d'autres types de pathologies telles que par exemple le traitement de troubles cardiovasculaires, du diabète, de la sclérose en plaques (SEP), ou encore celui de l'épilepsie.

Pour limiter ces risques liés à l'utilisation d'un dispositif médical logiciel, on connaît déjà le document WO 2014/125235 appartenant à la Demanderesse.

Ce document concerne ainsi un système informatique de « *matériovigilance* » impliquant la surveillance de l'ensemble des risques liés à la défaillance ou le mésusage de l'une des fonctionnalités proposées par le logiciel.

Le système proposé dans ce document vérifie en continu que le dispositif médical logiciel est utilisé dans les conditions et aux fins prévues à la fois par le fabricant mais également par le médecin.

Plus particulièrement, le système proposé dans ce document est configuré pour identifier de façon systématique tous risques logiciels, matériels et/ou informatiques liés au dispositif médical logiciel :
- une incompatibilité informatique ou logicielle,
- une mise à jour erronée,
- un système d'exploitation obsolète,
- une horloge désynchronisée,
- un problème d'interopérabilité,
- un modèle d'appareil auxiliaire non supporté,
- etc.

Le système proposé dans ce document est en outre configuré pour activer ou désactiver à distance le dispositif médical logiciel lorsqu'un risque a été identifié.

Le système proposé dans ce document cherche donc à éviter les risques logiciels, matériels et/ou informatiques qui résultent d'une défaillance ou d'un mésusage du dispositif médical logiciel.

Le document US 2014/0325065 A1 divulgue un système permettant d'activer une caractéristique d'une application mobile telle que prescrite par un médecin. Le médecin fournit au patient les données de prescription et un code d'activation qui doit être vérifié par un serveur afin d'activer l'application.

La Demanderesse soumet toutefois qu'il n'existe pas à ce jour de système qui cherche à vérifier que le dispositif médical logiciel fonctionne correctement en respectant les recommandations médicales du médecin.

En d'autres termes, aucune solution aujourd'hui n'est proposée aujourd'hui pour vérifier que le dispositif médical logiciel respecte la prescription médicale.

Dans une situation classique, la délivrance d'un produit de santé tel qu'un médicament ou un dispositif médical est encadrée pour éviter les risques d'accidents liés à ce produit de santé.

Le médecin, à la suite d'une consultation, établit donc sur une feuille une prescription médicale dans laquelle il indique le traitement à suivre pour le patient avec :
- le ou les médicaments et/ou,
- le ou les dispositifs médicaux associés à ce traitement par exemple un auto-tensiomètre pour le traitement d'une maladie comme l'hypertension artérielle.

Cette prescription peut donc contenir les informations relatives à la posologie du médicament, la dose recommandée, la période du traitement, les éventuelles contrindications médicales dues à des antécédents ou à la prise d'autres médicaments incompatibles, le mode d'utilisation du dispositif médical, les valeurs normales attendues pour une mesure réalisée avec le dispositif, etc.

Cette prescription contient également le numéro d'identification du médecin, le nom et le prénom du patient.

Le patient doit ensuite se munir de cette prescription pour aller par exemple chez un pharmacien et récupérer le ou les médicaments en question ainsi que le ou les dispositifs médicaux en question.

Une fois rentré chez lui, le patient doit suivre scrupuleusement les recommandations du médecin en prenant ses médicaments et/ou en utilisant le dispositif médical associé selon les recommandations figurant sur la prescription ; c'est-à-dire en respectant notamment la posologie, le dosage et la période de traitement ou encore le rythme et le mode d'utilisation du ou des dispositifs médicaux prescrits.

Dans le contexte des dispositifs médicaux logiciels, la situation est différente.

En effet, avec l'apparition des dispositifs médicaux logiciels, certaines caractéristiques du traitement, comme par exemple la détermination du dosage d'un médicament, sont réalisées automatiquement par des fonctionnalités logicielles spécifiques paramétrées en fonction des recommandations du médecin.

Une partie du traitement est donc dématérialisée et transférée dans les fonctionnalités logicielles du dispositif médical logiciel.

Cette nouvelle approche pour traiter le patient soulève de nombreux problèmes qui n'ont pas été solutionnés jusqu'à présent.

Un des problèmes techniques soulevés avec l'apparition de tels dispositifs médicaux logiciels est de trouver une solution pour prescrire une application logicielle à installer de la même façon qu'un médecin ou un professionnel de santé prescrirait un dispositif médical classique.

Une première solution pourrait être de noter sur une prescription, version papier, le nom de l'application logicielle à installer sur le terminal de communication servant de dispositif médical.

L'installation de l'application logicielle sur le terminal de communication du patient serait alors libre et se ferait via un serveur de téléchargement du type « *store* » tel que par exemple « *AppStore*® ».

Cette solution présenterait toutefois plusieurs désavantages :
- le patient peut perdre la prescription sur laquelle est noté le nom de l'application à installer ;
- le patient peut oublier d'installer l'application ;
- le patient peut ne pas savoir installer une telle application.

Un des autres problèmes techniques soulevés avec l'apparition des dispositifs médicaux logiciels est de trouver une solution pour s'assurer que le patient a correctement installé l'application sur son terminal de communication.

Une première solution pourrait être de demander au patient de confirmer par exemple par mail ou par SMS au médecin-prescripteur qu'il a bien installé l'application.

Cette solution présenterait toutefois de nombreux désavantages :
- le patient peut oublier de confirmer l'installation de l'application. Dans ce cas, le médecin ne sait pas si son patient a commencé le traitement prescrit ;
- le médecin-prescripteur doit endosser la responsabilité du suivi de son patient.

Un autre problème technique soulevé avec l'apparition des dispositifs médicaux logiciels est de trouver une solution pour établir le lien entre le patient et le médecin qui a prescrit l'application.

Pour cela, il faudrait d'abord prévoir une solution pour vérifier que la prescription a bien été émise par un médecin.

Ensuite, on pourrait par exemple demander au médecin, après avoir prescrit l'application à son patient, de l'enregistrer sur le serveur hébergeant l'application. Une fois l'application installée, le patient saisirait alors son nom, son prénom et sa date de naissance pour se connecter à l'application, lors de sa première connexion au serveur.

Lors de sa prochaine synchronisation, le patient serait ensuite identifié sur le serveur *Web* grâce aux informations qu'il a saisies lors de sa première connexion.

Cette solution présenterait de nombreux désavantages : si par exemple le médecin se trompe dans l'orthographe du nom et/ou du prénom, ou dans la date de naissance du patient, le lien ne pourrait jamais se faire.

Une solution alternative serait d'utiliser une information d'identification unique pour identifier le patient. Toutefois, dans de nombreux contextes, cette information n'existe pas ou n'est pas disponible : on sait par exemple que le numéro de Sécurité Sociale existe en France mais son utilisation est très réglementée et ne garantit pas l'unicité de l'individu.

Un des autres problèmes techniques soulevés avec l'apparition des dispositifs médicaux logiciels est de trouver une solution pour empêcher le patient d'utiliser son application sans validation du médecin.

Cette question est très importante sur le plan médical notamment pour assurer la sécurité du patient.

C'est le cas par exemple d'une application logicielle nécessitant une configuration et un paramétrage spécifiques adaptés au patient.

Un tel paramétrage est nécessaire par exemple pour le dosage spécifique d'un médicament, ou pour les règles d'auto-dosage de ce médicament par le patient.

De façon générale, il faut s'assurer que le paramétrage du dispositif médical logiciel est conforme aux recommandations du médecin.

Une solution pour remédier à cet autre problème serait par exemple que le médecin demande (par message électronique du type mail ou SMS, ou par téléphone) au patient d'attendre sa confirmation avant d'utiliser l'application mobile, et que le médecin paramètre lui-même l'application une fois celle-ci installée.

Cette solution présenterait toutefois plusieurs désavantages :
- le patient peut ne pas lire ses messages électroniques ou ne pas avoir donné le bon numéro de téléphone au médecin. Dans ce cas, il ne peut pas recevoir l'information lui demandant d'attendre l'utilisation de l'application ; il peut alors faire un mauvais usage de l'application et obtenir par exemple un mauvais dosage de médicament donné par défaut par l'application ;
- le patient peut également être tenté d'utiliser l'application (par curiosité) sans attendre la confirmation du médecin et en faire dans ce cas un mauvais usage.

Une telle solution est par ailleurs contraignante pour le médecin.

En tout état de cause, aucune des solutions envisagées jusqu'à présent dans l'état de la technique ne propose un système informatique simple à déployer, limitant les interactions entre médecin et patient, et permettant de limiter les risques liés à une mauvaise utilisation d'une ou plusieurs fonctionnalités logicielles mises en oeuvre par un dispositif médical logiciel.

### Objet et Résumé de la présente invention

La présente invention vise à améliorer la situation décrite ci-dessus.

La présente invention s'inscrit dans une même démarche sécuritaire que le document WO 2014/125235 et vise à remédier aux différents inconvénients mentionnés ci-dessus en s'assurant que le paramétrage des fonctionnalités logicielles pour accompagner le patient dans son traitement est rigoureusement conforme à la prescription médicale établie par le médecin spécifiquement pour son patient.

A cet effet, l'objet de la présente invention concerne selon un premier aspect un procédé d'activation d'une application logicielle apte à mettre en oeuvre sur un terminal de communication des fonctionnalités logicielles pour accompagner un patient dans un traitement thérapeutique déterminé.

Selon l'invention, le terminal de communication sert ici de dispositif médical logiciel.

Dans le cadre de la présente invention, le traitement thérapeutique que doit suivre le patient est déterminé selon une prescription médicale informatisée.

Cette prescription médicale informatisée est stockée dans une base de données connectée à un serveur central distant.

Les fonctionnalités logicielles mises en oeuvre par l'application logicielle sur le terminal de communication doivent donc suivre cette prescription.

Il s'agit de fonctionnalités logicielles permettant par exemple :
- le dosage d'un bronchodilatateur pour le traitement de l'asthme,
- le suivi de l'observance du traitement contre certaines maladies auto-immunes,
- le dosage d'un anticoagulant pour le traitement de certains troubles cardiaques,
- le dosage en carbamazépine pour le traitement de l'épilepsie,
- etc.

Bien évidemment, ces exemples de fonctionnalités ne présentent en aucun cas un caractère limitatif et sont purement illustratifs ; l'homme du métier comprend ici que d'autres fonctionnalités relevant du domaine du dispositif médical peuvent être envisagées dans le cadre de la présente invention pour accompagner un patient dans le traitement d'autres pathologies.

Cette prescription médicale informatisée comprend de façon caractéristique des données de prescription personnelles contenant des informations associées au traitement prescrit au patient.

De préférence, les données de prescription personnelles comprennent notamment des informations relatives :
- au patient,
- au praticien, et/ou
- au médicament prescrit par le praticien pour le traitement du patient.

Ces données de prescription peuvent également contenir des informations relatives au dispositif médical ; il peut s'agir par exemple d'informations concernant notamment :
- le dosage dudit médicament prescrit, et/ou
- les règles d'auto-dosage de ce médicament par le patient lui-même, et/ou
- la posologie associée, et/ou
- les valeurs normales de mesure par un dispositif médical associé.

Ces données de prescription sont destinées à être exécutées et/ou utilisées pour la mise en oeuvre d'au moins une des fonctionnalités logicielles de l'application logicielle installée sur le terminal de communication.

Ces données peuvent également inclure des caractéristiques techniques du dispositif médical logiciel et/ou des paramètres de configuration des fonctionnalités logicielles.

Ces données de prescription sont caractéristiques de la présente invention.

Le procédé est avantageusement mis en oeuvre par des moyens informatiques et comporte les étapes suivantes :
- suite à une installation de l'application sur le terminal de communication, une étape d'authentification du patient auprès du serveur central via le terminal de communication ;
- lorsque le patient est authentifié auprès du serveur central, une étape d'activation à distance de l'application et des données de prescription sur le terminal de communication pour autoriser la mise en oeuvre d'au moins une des fonctionnalités logicielles selon les données de prescription sur le terminal.
- l'étape d'activation comprenant une transmission à destination du terminal de communication d'un message d'activation comprenant les données de prescription et un ordre d'activation.

La présente invention consiste donc en :
- une authentification du patient à l'aide du terminal de communication sur lequel est installée l'application avec un serveur central stockant la prescription médicale informatisée, puis
- une activation de l'application et des données de prescription par la transmission à destination du terminal de communication d'un message d'activation comprenant les données de prescription et un ordre d'activation pour permettre l'exécution et la mise en oeuvre d'au moins une des fonctionnalités logicielles de l'application selon les données de prescription sur le terminal de communication.

Cette succession d'étapes techniques, caractéristique de la présente invention, permet de garantir que les fonctionnalités mises en oeuvre sur le terminal de communication respectent les recommandations médicales définies par le médecin pour son patient dans la prescription.

Ainsi, grâce à cette authentification et cette activation gérées par un serveur central distant, la fonctionnalité servant par exemple pour calculer le dosage d'un médicament réalise ce calcul en fonction des données de prescription.

Dans la mesure où ces données sont personnelles et sont adaptées au traitement du patient, le dosage est calculé spécifiquement pour le patient selon les recommandations définies par le médecin, et non en fonction d'un calcul de dosage pris par défaut par l'application dans sa version téléchargeable (non personnalisée).

On peut prévoir que l'étape d'authentification comprend également l'authentification du terminal en tant que tel. Dans ce cas, cette authentification du terminal se fait en vérifiant un identifiant propre au terminal. Cette authentification supplémentaire permet de vérifier que le patient utilise bien le terminal autorisé (par exemple celui recommandé par le médecin ou celui prêté par le médecin).

Dans un mode de réalisation avantageux, l'étape d'authentification comprend la comparaison par le serveur central d'un code reçu du terminal de communication avec un code unique de prescription enregistré dans la base de données.

Dans ce mode, on peut prévoir par exemple que le patient s'est authentifié auprès du serveur central si les deux codes comparés sont similaires ; c'est-à-dire ici si le code provenant du terminal et reçu par le serveur contient effectivement l'information d'identification contenue dans le code unique de prescription.

Un tel code unique de prescription est optionnel.

De préférence, le procédé selon la présente invention comprend une étape de génération du code unique de prescription en fonction des données de prescription.

Ce code peut également être généré en fonction des données de prescription et d'un numéro unique d'identification.

Ce numéro unique d'identification est généré par le médecin à partir des informations personnelles relatives au patient et des informations d'identification permettant de communiquer avec le terminal de communication : par exemple un numéro de téléphone ou une adresse électronique.

Ce numéro unique d'identification permet donc d'identifier le patient et de communiquer avec lui par l'intermédiaire de son terminal.

La prescription du patient est donc validée puis activée en utilisant ce code unique de prescription. Ce code unique de prescription permet donc d'authentifier la prescription avant activation.

Le Demandeur soumet qu'on pourrait également prévoir un mode de fonctionnement, où le médecin va aussi authentifier le terminal lui-même. Typiquement, il s'agit du cas d'un contexte d'étude clinique où un terminal est prêté au patient. Dans ce cas, il y aura une étape d'authentification du terminal pour s'assurer que le patient ne peut utiliser l'application que sur le terminal qui a été fourni au patient.

Avantageusement, le code unique de prescription se présente sous la forme d'un code matriciel à deux dimensions.

De préférence, ce code est un code du type « *Flash Code*® » ou « *QR Code*® ».

Ce code peut s'afficher par exemple sur l'écran de l'ordinateur du médecin ou encore peut être apposé sur des coupons prédéfinis servant de prescription.

De préférence, ce code matriciel à deux dimensions contient des informations relatives aux paramètres de configuration des fonctionnalités logicielles mises en oeuvre sur ledit dispositif médical.

Avantageusement, le procédé selon la présente invention comprend une étape de lecture optique du code unique de prescription, par exemple par des moyens de lecture optique du terminal de communication.

On comprend donc ici que le patient peut lire le code matriciel avec son terminal de communication directement à partir de l'écran d'ordinateur du médecin ou à partir du coupon donné au patient par le médecin et servant de prescription.

Dans un mode de réalisation avantageux, le procédé selon la présente invention comprend, suite à l'authentification, une étape d'émission au cours de laquelle le serveur central émet un code d'accès à durée limité.

Ce code d'accès est émis à destination du terminal de communication.

Alternativement, celui-ci peut être émis à un autre terminal appartenant au patient.

Ce code permet avantageusement d'autoriser l'activation de l'application et des données de prescription.

Ceci est avantageux par exemple :
- lorsque le patient ne souhaite pas installer tout de suite l'application, et qu'il préfère procéder à l'installation une fois rentré chez lui, ou
- lorsque le patient ne dispose pas de son terminal de communication lors de la consultation ou qu'il n'a pas accès au réseau de données pour se connecter au serveur.

Dans ce mode, on peut prévoir par exemple que le code d'accès à durée limitée est émis dans un message du type SMS ou courriel.

Le Demandeur soumet qu'il est possible d'envisager l'envoi seul du code d'accès à durée limitée ; cet unique code permet alors d'authentifier le médecin.

Ceci peut correspondre au cas dans lequel le médecin n'a pas besoin de matérialiser sa prescription au patient par un document spécifique.

Dans ce cas, le médecin programme alors les informations relatives au patient ; le patient reçoit alors un message qui ne contient qu'un seul code (le code d'accès usage unique) qui permettra de garantir l'origine de la prescription et son destinataire.

La vérification du code de prescription unique couplée éventuellement à la validation du code d'accès à durée limitée permettent d'associer le compte du patient au médecin qui le suit ; plus particulièrement, la vérification du code de prescription unique permet une garantie de l'origine (c'est-à-dire le médecin prescripteur), et la validation du code d'accès à durée limitée permet de garantir le destinataire (le patient auquel l'application a été prescrite).

Le Demandeur soumet que l'avantage de ces deux codes permet au patient d'avoir, à sa sortie du cabinet du médecin, une trace physique de la prescription qui lui a été faite.

Corrélativement, l'objet de la présente invention concerne selon un deuxième aspect un programme d'ordinateur.

Selon l'invention, ce programme comporte des instructions adaptées pour l'exécution des étapes du procédé tel que décrit ci-dessus, ceci notamment lorsque ledit programme d'ordinateur est exécuté par au moins un processeur ou un ordinateur.

Un tel programme d'ordinateur peut utiliser n'importe quel langage de programmation, et être sous la forme d'un code source, d'un code objet, ou d'un code intermédiaire entre un code source et un code objet, tel que dans une forme partiellement compilée, ou dans n'importe quelle autre forme souhaitable.

De même, l'objet de la présente invention concerne selon un troisième aspect un support d'enregistrement.

Ce support est lisible par un processeur ou ordinateur sur lequel est enregistré un programme d'ordinateur comprenant des instructions pour l'exécution des étapes du procédé tel que décrit ci-dessus.

D'une part, le support d'enregistrement peut être n'importe quel entité ou dispositif capable de stocker le programme. Par exemple, le support peut comporter un moyen de stockage, tel qu'une clé USB ou tel qu"une mémoire ROM, par exemple un CD-ROM ou une mémoire ROM de type circuit microélectronique, ou encore un moyen d'enregistrement magnétique tel que par exemple un disque dur.

D'autre part, ce support d'enregistrement peut être un support transmissible tel qu'un signal électrique ou optique, un tel signal pouvant être acheminé via un câble électrique ou optique, par radio classique ou hertzienne ou par faisceau laser autodirigé ou par d'autres moyens. Le programme d'ordinateur selon l'invention peut être en particulier téléchargé sur un réseau de type Internet.

Alternativement, le support d'enregistrement peut être un circuit intégré dans lequel le programme d'ordinateur est incorporé, le circuit intégré étant adapté pour exécuter ou pour être utilisé dans l'exécution du procédé en question.

L'objet de la présente invention concerne selon un quatrième aspect un système informatique d'activation d'une application logicielle apte à mettre en oeuvre sur un terminal de communication des fonctionnalités logicielles.

Selon l'invention, les fonctionnalités logicielles sont ici prévues pour accompagner un patient dans un traitement thérapeutique déterminé selon une prescription médicale informatisée ; cette prescription comprend des données de prescription personnelles contenant les informations associées au traitement prescrit au patient.

Avantageusement, le système d'activation selon l'invention comporte des moyens informatiques configurés pour la mise en oeuvre des étapes du procédé tel que décrit ci-dessus.

Plus particulièrement, le système d'activation comprend notamment:
- une base de données stockant la prescription médicale informatisée ;
- des moyens d'authentification aptes à coopérer avec des premiers moyens de communication pour authentifier le patient via le terminal de communication sur lequel est installée l'application;
- des moyens d'activation aptes à coopérer avec des premiers moyens de communication pour activer à distance l'application et les données de prescription sur le terminal de communication afin d'autoriser la mise en oeuvre d'au moins une desdites fonctionnalités logicielles selon les données de prescription sur le terminal, lesdits moyens d'actvation étant aptes à transmettre à destination du terminal de communication un message d'activation comprenant les données de prescription et un ordre d'activation

L'objet de la présente invention porte également selon un cinquième aspect sur un serveur informatique comportant un système d'activation tel que décrit ci-dessus.

L'objet de la présente invention porte enfin selon un sixième aspect sur un dispositif médical, dit logiciel, comportant un terminal de communication sur lequel est installée une application logicielle apte à mettre en oeuvre des fonctionnalités logicielles pour accompagner un patient dans un traitement thérapeutique déterminé selon une prescription médicale informatisée.

Cette prescription, stockée dans une base de données connectée à un serveur central distant, comprend des données de prescription personnelles contenant des informations associées au traitement prescrit au patient.

Ce dispositif médical selon l'invention comporte :
- un circuit électronique d'authentification configuré pour une authentification du patient auprès du serveur central via le terminal ; et
- un circuit électronique d'activation configuré pour activer l'application et les données de prescription sur le terminal de communication pour autoriser la mise en oeuvre selon les données de prescription d'au moins une desdites fonctionnalités logicielles sur le terminal sur réception d'un message d'activation comprenant les données de prescription et un ordre d'activation.

Dans un mode de réalisation avantageux, le circuit d'authentification est apte à coopérer avec des deuxièmes moyens de communication pour émettre à destination du serveur central une requête comprenant un code à comparer avec un code unique de prescription enregistré dans la base de données.

Ainsi, l'objet de la présente invention, par ses différents aspects fonctionnels et structurels décrits ci-dessus, permet l'activation à distance d'un dispositif médical logiciel pour garantir que la mise en oeuvre des fonctionnalités logicielles est conforme à une prescription médicale informatisée établie par le médecin spécifiquement pour le traitement du patient.

### Brève description des figures annexées

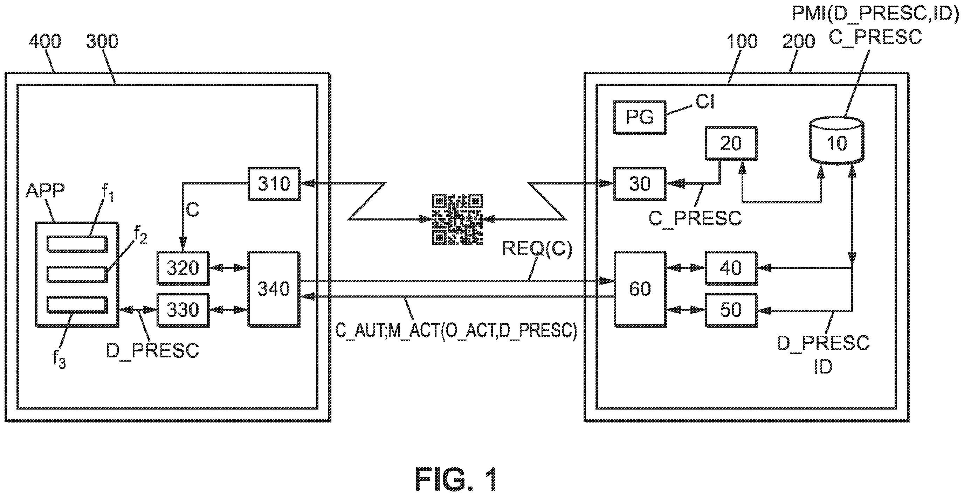

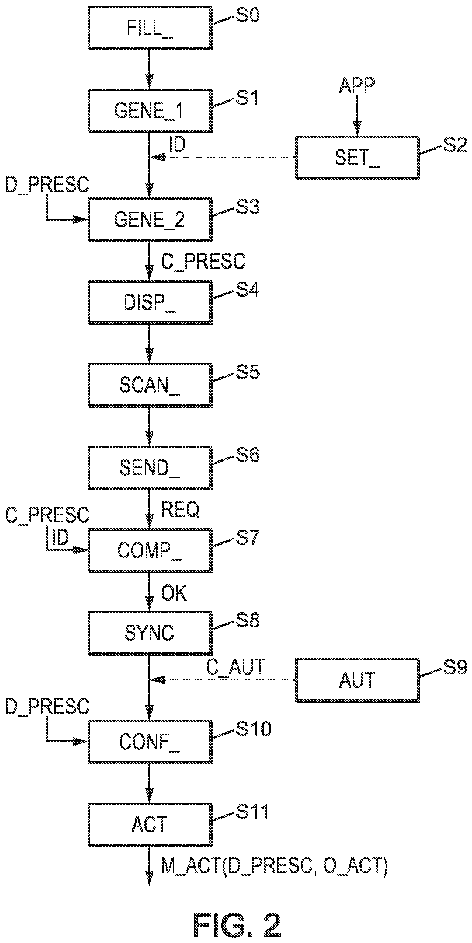

D'autres caractéristiques et avantages de la présente invention ressortiront de la description ci-dessous, en référence aux figures 1 et 2 annexées qui en illustrent un mode de réalisation dépourvu de tout caractère limitatif et sur lesquelles :
- la figure 1 représente de façon schématique un mode de mise en oeuvre selon la présente invention avec un serveur comprenant un système informatique d'activation à distance d'une application logicielle apte à mettre en oeuvre sur un terminal de communication des fonctionnalités logicielles pour accompagner un patient dans un traitement thérapeutique déterminé ; et
- la figure 2 représente un organigramme illustrant le procédé d'activation conforme à un exemple de réalisation de la présente invention (premier scénario - cas 1).

### Description détaillée de différents exemples de mise en oeuvre

Une activation à distance d'une application logicielle APP mettant en oeuvre au moins une des fonctionnalités logicielles f1, f2, et f3 sur un terminal de communication 300 pour accompagner un patient dans un traitement thérapeutique va maintenant être décrite dans ce qui va suivre en faisant référence conjointement aux figures 1 et 2.

Pour traiter l'hypertension artérielle, le patient doit prendre un médicament du type Inhibiteur de l'Enzyme de Conversion, noté ci-après IEC.

Ce médicament nécessite une adaptation régulière de la posologie et du dosage, en fonction notamment de la tension artérielle systolique du patient et de données personnelles relatives au patient.

L'adaptation de cette posologie et du dosage de l'IEC doit donc être déterminée par un médecin lors d'une consultation en fonction d'informations physiologiques et personnelles du patient recueillies par le médecin lors d'une consultation :
- l'âge du patient,
- son poids,
- ses antécédents médicaux,
- sa tension artérielle systolique de référence,
- ses variations de tension,
- etc.

Cette posologie et ce dosage sont spécifiques au patient.

On comprendra ici qu'il n'est pas possible d'envisager la prise d'un médicament du type IEC sans avis médical, ceci pour des raisons évidentes liées à la santé du patient.

Bien évidemment, comme expliqué précédemment, l'exemple du traitement de l'hypertension est purement illustratif et ne présente en aucun cas un caractère limitatif : l'homme du métier comprend ici que le patient peut être accompagné dans le traitement de tout autre type de pathologie avec un dispositif médical selon la présente invention.

En effet, l'exemple décrit ici peut être transposé sans aucune difficulté au traitement d'une autre pathologie telle que par exemple l'asthme, la SEP, le diabète, etc.

Comme n'importe quel médicament, un médicament du type IEC doit être pris selon les recommandations d'un médecin (c'est-à-dire en fonction d'une prescription médicale) : la prise d'un tel médicament suite à un dosage par défaut peut être dangereuse.

Dans l'exemple décrit ici, on dispose d'un terminal de communication 300 ; ce terminal sert de support au dispositif médical logiciel 400.

Dans cet exemple, le dispositif médical logiciel 400 dont il est question doit permettre l'accompagnement thérapeutique d'un patient souffrant d'hypertension artérielle.

Un tel dispositif médical 400 est donc prévu pour mettre en oeuvre une ou plusieurs des fonctionnalités logicielles f1, f2, et f3 visant à déterminer de façon dynamique et personnalisée le dosage d'IEC.

Il est important pour la santé du patient que ces fonctionnalités logicielles f1, f2 et f3 mises en oeuvre sur le terminal de communication 300 respectent la prescription établie par le médecin pour accompagner correctement le patient dans son traitement thérapeutique.

Permettre l'activation à distance d'une application logicielle APP et d'une prescription médicale informatisée PMI sur un terminal de communication 300 afin de respecter les recommandations du médecin est un des objectifs de la présente invention.

La présente invention va être décrite plus en détails selon deux *scénarii.*

### Premier scénario :

Dans ce premier scénario, il est considéré au préalable que le médecin qui suit le patient dispose lors de la consultation d'une connexion au réseau 3G et d'un accès à un serveur central 200 accessible via un portail *Web* dédié.

De même, il est considéré dans ce premier scénario que le patient dispose lors de cette consultation d'un terminal de communication 300 tel qu'un « *Smartphone* » ou une tablette numérique, avec un accès à un réseau de communication par exemple un accès au réseau 3G.

Dans cet exemple, le patient souffrant d'hypertension artérielle vient donc en consultation chez son médecin pour se faire prescrire une application logicielle APP.

Cette application APP est dédiée à l'accompagnement du patient dans le traitement de l'hypertension artérielle.

Plus particulièrement, dans cet exemple, l'application logicielle APP dont il est question est apte à mettre en oeuvre des fonctionnalités logicielles f1, f2, et f3 permettant notamment le calcul automatique d'un médicament du type IEC pour prévenir, retarder ou limiter, voire supprimer les risques liés à l'hypertension.

Cette application APP est donc destinée à être installée sur le terminal de communication 300 du patient.

S'assurer que le calcul du dosage du médicament de type IEC par le terminal 300 est bien déterminé selon la prescription médicale établie par le médecin pour le patient est un des objectifs de la présente invention.

Ceci est rendu possible dans le cadre de la présente invention par un système informatique d'activation 100.

Ce système 100 est accessible via le serveur informatique 200.

En d'autres termes, le serveur 200 héberge un tel système 100.

Selon ce premier scénario, le médecin se connecte en début de consultation à ce serveur 200 via le portail *Web* dédié ; il renseigne lors d'une première étape S0 une base de données 10 du système 100 avec des informations personnelles relatives au patient, par exemple :
- son nom,
- son prénom,
- sa date de naissance, et
- son numéro de téléphone et/ou son adresse mail.

Toute autre combinaison d'informations permettant d'identifier le patient de manière fiable peut également être enregistrée dans cette base de données 10 (par exemple le numéro de Sécurité Sociale).

Lors d'une étape S1, les moyens informatiques de génération 20 du système 100 génèrent à partir de ces informations un numéro unique d'identification ID.

Ce numéro ID est personnel ; il est associé au patient.

Une fois ce numéro ID généré, le médecin demande au patient s'il souhaite installer l'application APP prescrite ; il a le choix entre réaliser cette installation immédiatement (cas 1) ou l'installer plus tard (cas 2), par exemple lorsqu'il rentre chez lui.

Si le patient souhaite installer immédiatement l'application APP sur son terminal 300, il a un délai déterminé pour le faire (par exemple 15 minutes) ; ce délai permet au médecin d'attester de l'identité du patient et éventuellement de paramétrer le modèle de prescription si nécessaire.

Passé ce délai (une fois rentré à son domicile par exemple), le patient ne peut installer son application APP qu'avec un code d'accès qui lui sera envoyé soit par SMS, soit par mail.

### Cas 1 :

Dans ce premier cas, le patient décide d'installer l'application logicielle APP sur son terminal de communication 300 dans le délai imparti.

Lors d'une étape S2, le patient installe donc cette application logicielle APP sur son terminal 300 à partir d'un « *Store* » dédié, ou d'un « *Store* » accessible au public.

On comprend ici que ce « *Store* » peut être directement associé au serveur 200.

Alternativement, il peut s'agir d'un serveur distinct, spécialement dédié au téléchargement.

Dans cet exemple, le médecin génère ensuite un code unique de prescription C_PRESC.

Cette génération est réalisée lors d'une étape S3 par les moyens de génération 20 du système 10.

Dans l'exemple décrit ici, ce code C_PRESC peut résulter d'une agrégation des données personnelles relatives au patient, par exemple le numéro unique d'identification ID, et des données de prescription D_PRESC qui sont relatives à la prescription en tant que telle.

Plus particulièrement, ce code unique de prescription C_PRESC contient dans l'exemple décrit ici les informations suivantes :
- la date de la prescription,
- le numéro unique d'identification ID,
- le modèle de prescription contenant les médicaments prescrits, leur dosage (éventuellement configurable), et leur posologie (éventuellement configurable),
- l'identifiant unique du médecin, et
- le nom et le prénom du médecin.

Le modèle de prescription est associé à la pathologie et au médicament prescrit.

Il comprend donc des champs pré-remplis correspondants au médicament, son dosage, sa posologie, etc.

Les valeurs associées à ces champs peuvent être configurables, par exemple si les valeurs prises par défaut ne sont pas adaptées au patient ou si elles nécessitent un paramétrage spécifique pour le patient.

L'ensemble de ces informations forme la prescription médicale informatisée PMI.

Cette prescription PMI associée à ce code C_PRESC est stockée dans la base de données 10 du système 100.

Cette prescription PMI et ce code C_PRESC peuvent également être chiffrés. De préférence, le mécanisme de chiffrement utilisé est un mécanisme de clef privée/clef publique pour améliorer la sécurité des informations qu'ils contiennent.

La base de données 10 peut également être intégrée dans un module sécurisé.

Le code unique de prescription C_PRESC est caractéristique de la présente invention.

L'exécution des informations contenues dans ce code C_PRESC, et plus particulièrement l'exécution des données de prescription D_PRESC, vont permettre de mettre en oeuvre au moins une des fonctionnalités logicielles f1, f2, et f3 de l'application APP conformément à la prescription médicale PMI du médecin.

De préférence, ce code C_PRESC peut se présenter sous la forme d'un code matriciel à deux dimensions tel que par exemple un « *QR Code* » ou un « *Flash Code* ».

Dans l'exemple décrit ici, ce code matriciel s'affiche sur l'ordinateur du médecin lors d'une étape S4.

Lors d'une étape S5, le patient scanne (lecture optique) alors ce code à l'aide des moyens de lecture optique 310 de son terminal de communication 310.

Alternativement, le patient peut saisir manuellement ce code C_PRESC dans l'application APP qu'il vient d'installer.

Dans un cas comme dans l'autre, un message s'affiche alors sur le terminal 300 du patient lui indiquant que le médecin lui a prescrit cette application APP.

Pour pouvoir utiliser l'application APP, il faut s'assurer que celle-ci est correctement paramétrée en fonction des recommandations du médecin ; c'est-à-dire en fonction de la prescription médicale informatisée PMI.

Le patient authentifie donc le terminal de communication 300 sur lequel est installée l'application APP auprès du système d'activation 100 afin de vérifier que le code C scanné ou saisi par le patient est correct.

Cette étape d'authentification comprend donc une comparaison S7 du code C scanné ou saisi par le patient sur son terminal 300 avec le code de prescription C_PRESC généré sur le système 100.

Pour réaliser cette comparaison S7, le terminal de communication 300 comprend un circuit électronique d'authentification 320 qui coopère lors d'une étape S6 avec des deuxièmes moyens de communication 340 pour émettre à destination du serveur central 200 une requête REQ comprenant le code C.

Le système 100 reçoit cette requête REQ via les moyens de communication 60 ; les moyens d'authentification 40 interrogent ensuite la base de données 10 pour comparer lors de cette étape S7 le code C reçu avec le code unique de prescription C_PRESC.

Plus particulièrement, dans l'exemple décrit ici, ce code C est comparé au numéro d'identification unique ID contenu dans le code C_PRESC.

Si les deux codes sont identiques, le terminal 300 et le système d'activation 100 se synchronisent lors d'une étape S8.

Le système d'activation 100 renvoie alors au terminal 300 les nom et prénom du patient associé à ce code unique de prescription C_PRESC.

Optionnellement, le patient peut vérifier l'orthographe, et corriger le cas échéant.

Si le modèle de prescription contenu dans les données de prescription D_PRESC est configurable, le médecin peut également configurer ce modèle avec si nécessaire des paramètres propres au patient. Ceci est réalisé lors d'une étape S10.

Lors de cette étape, le médecin modifie et/ou complète les données de prescription D_PRESC contenues dans la base de données 10.

Une fois configurée, il est prévu dans l'exemple décrit ici une étape d'activation S11 au cours de laquelle les moyens d'activation 50 émettent via les premiers moyens de communication 60 un message d'activation M_ACT à destination du terminal 300.

Ce message M_ACT comprend un ordre d'activation O_ACT et les données de prescription D_PRESC.

Sur réception de ce message M_ACT, le circuit électronique d'activation 330 déclenche l'activation sur le terminal de communication 300 de l'application APP et des données de prescription D_PRESC.

Le patient peut alors utiliser l'application APP sans risque pour sa santé ; les fonctionnalités logicielles f1, f2 et f3 mises en oeuvre sont conformes à la prescription médicale PMI établie par le médecin spécifiquement pour le patient.

### Cas 2 :

Dans ce deuxième cas, le patient décide d'installer l'application plus tard (ou le délai imparti pour l'installation est dépassé).

Lors de la consultation, le médecin alimente la base de données 10 comme dans le premier cas.

Il génère un code de prescription C_PRESC.

Le patient rentre chez lui et installe l'application APP sur son terminal 300. Il lance alors l'application APP qu'il vient d'installer.

Dans cet exemple, il est prévu de façon optionnelle une authentification du patient.

Ainsi, optionnellement, après avoir vérifié lors de l'authentification que le code unique de prescription C_PRESC est valide, le serveur 200 envoie un code d'accès C_AUT à durée de vie limitée (par exemple 48 heures) au patient soit par SMS, soit par courriel.

Le patient muni de son terminal saisit le code d'accès C_AUT reçu dans la zone prévue à cet effet.

Ensuite, le patient s'authentifie à l'aide du terminal 300 auprès du serveur 200 afin de vérifier que le code d'accès est valide.

Une fois validé, on retrouve les mêmes étapes que dans le premier cas de figure décrit ci-dessus.

Le Demandeur soumet qu'il est possible que le code d'accès C_AUT ne débloque qu'une partie des fonctionnalités de l'application.

Par exemple, on peut envisager que, sur la base du code unique de prescription C_PRESC, le patient peut n'avoir accès qu'aux fonctionnalités f1 et f3 et, qu'après vérification du code d'accès C_AUT, le patient ait accès à f2.

Ainsi, dans un exemple particulier, on peut prévoir que le patient entre lui-même sa tension, son pouls ou tout autre paramètre médical, et que la fonctionnalité d'adaptation de son dosage de médicament ne deviendrait disponible qu'après « activation finale » en utilisant le code d'accès C_AUT.

Dans cet exemple, le mécanisme utilisant ce code C_AUT a pour objectif d'éviter de donner des conseils médicaux inadaptés (dont certains pourraient être fatals) à un mauvais patient.

Le Demandeur soumet qu'il est possible d'envisager un mode de réalisation dans lequel le médecin alimente la base de données 10 avec les informations requises et que le code d'accès C_AUT à durée de vie limitée soit envoyé directement sans que le code unique de prescription C_PRESC ne soit généré.

On comprend donc dans ce cas que la génération du code C_PRESC est optionnelle.

### Deuxième scénario :

Dans ce deuxième scénario, il est considéré au préalable que le médecin n'a pas accès au serveur *Web,* et que le patient vient à la consultation sans son terminal de communication 300 ou qu'il n'a pas accès à un réseau de communication.

Comme dans le premier scénario, le patient vient ici en consultation chez son médecin pour se faire prescrire une application APP.

Dans cet exemple de mise en oeuvre, le médecin dispose d'un carnet de prescriptions d'application mobile (avec copies carbone) pour chaque modèle de prescription.

Chaque prescription dispose d'un code unique de prescription C_PRESC stocké dans la base de données 10 du serveur *Web* 200 et associé à un professionnel de santé.

Chaque prescription contient donc :
- le code unique de prescription C_PRESC (uniquement pour la copie carbone) ;
- un modèle de prescription contenant le ou les médicaments prescrits, leur dosage (éventuellement configurable), leur posologie (éventuellement configurable) et/ou le ou les dispositifs médicaux prescrits ainsi que les caractéristiques associées ;
- l'identifiant unique du médecin ;
- le nom et le prénom du médecin.

Toute autre combinaison d'informations permettant d'identifier de façon unique la prescription peut également convenir ici.

Sur cette prescription, plusieurs champs permettent également d'écrire :
- le nom et le prénom du patient,
- la date de naissance du patient,
- le ou les numéros de téléphone du patient,
- l'adresse électronique du patient,
- l'adresse postale du patient.

On notera ici qu'une prescription peut ne pas comporter de médicament, mais uniquement un voire plusieurs dispositifs médicaux et ses caractéristiques.

Prenons l'exemple d'un ophtalmologue qui prescrit une paire de lunettes : il inscrit sur l'ordonnance les facteurs correctifs des verres à donner au patient, ces informations correspondent ici aux caractéristiques du dispositif médical.

De la même façon, dans le cadre du diabète, une prescription peut par exemple comporter un glucomètre configuré pour fonctionner dans une certaine unité, une pompe à insuline avec des caractéristiques concernant les débits d'insuline, etc.

Lors de la consultation, le médecin choisit dans son carnet de prescriptions le modèle correspondant à l'application mobile à prescrire au patient.

Il remplit ensuite les champs de la prescription avec les informations fournies par le patient.

Le médecin donne ensuite au patient la prescription.

Le patient rentre chez lui.

### Cas 1 :

Le médecin enregistre le patient sur le serveur *Web* 200 ; il lui associe le code unique de prescription C_PRESC (grâce à la copie carbone de la prescription) avant que le patient n'ait installé l'application APP sur son terminal 300.

Le serveur *Web* 200 envoie un message au patient via un des canaux disponibles sur le serveur 200 et selon les informations connues du patient:
- par SMS si le numéro de téléphone mobile est connu,
- par serveur vocal si seul le numéro de téléphone fixe est connu, ou encore
- par mail si l'adresse électronique du patient est connue, etc.),
pour lui indiquer qu'il peut désormais installer l'application APP sur son terminal 300.

Le patient installe donc l'application sur son terminal 300 à partir du « *Store* » dédié.

Le patient scanne ensuite le code se trouvant sur la prescription ou le saisit manuellement dans l'application APP.

Un message s'affiche sur le terminal 300 indiquant au patient que le médecin lui a prescrit cette application APP.

On retrouve ensuite les mêmes étapes que précédemment.

Le patient authentifie son terminal 300 auprès du serveur 200 afin de vérifier que le code unique de la prescription C_PRESC existe et est connu de la base de données 10.

Après avoir vérifié que ce code est valide, un code d'accès C_AUT (permettant d'activer l'application) à durée de vie limitée (48h) est envoyé au patient soit par SMS, soit par mail.

Le patient lance alors l'application APP qu'il a installée sur son terminal et saisit le code d'accès C_AUT reçu dans la zone prévue à cet effet.

Si le patient n'a pas activé l'application APP dans les 48h, il peut demander au médecin de lui générer un nouveau code d'accès et de lui envoyer.

Il est ensuite contrôlé que le code d'accès est valide.

Si tel est le cas, le serveur renvoie alors les nom et prénom du patient associé à ce numéro unique de prescription.

Le patient peut les vérifier et les corriger le cas échéant.

Comme dans les autres situations décrites ci-dessus, si le modèle de prescription est configurable, le professionnel de santé peut le configurer sur le serveur web avec des paramètres propres au patient.

A la suite de ces différentes étapes, le patient peut utiliser l'application APP sans risque.

### Cas 2 a:

Il est tout à fait possible d'envisager un autre cas dans lequel le patient installe l'application APP sur son terminal 300 alors que le médecin ne l'a pas encore inscrit sur le serveur *Web.*

Dans ce cas, le patient installe donc l'application APP à partir de son « *Store* ».

Le patient scanne le code C_PRESC se trouvant sur sa prescription ou le saisit manuellement dans l'application.

Un message s'affiche alors sur le terminal 300 du patient lui indiquant que le médecin lui a prescrit cette application APP.

Le patient s'authentifie auprès du serveur 200 afin de vérifier que le code unique de la prescription C_PRESC existe.

Dans ce cas, le serveur 200 ne trouve pas de patient associé au code unique de la prescription.

Le médecin associé au code unique de prescription est alors informé que le patient à qui, il a prescrit l'application APP, vient d'effectuer l'installation sur son terminal 200, mais qu'il ne peut pas l'utiliser car il n'est pas connu du serveur *Web* 200.

Le médecin inscrit dans ce cas le patient sur le serveur 200 et lui associe le code unique de prescription (grâce à la copie carbone de la prescription).

Si le modèle de prescription est configurable, le médecin peut le configurer sur le serveur 200 avec des paramètres propres au patient.

Le serveur *Web* 200 envoie ensuite un message au patient pour lui indiquer qu'il peut désormais synchroniser l'application.

Le patient synchronise l'application avec le serveur web afin de vérifier que le numéro unique de la prescription existe.

On retrouve ensuite les étapes précédemment décrites.

On comprendra ici que, quel que soit le mode de réalisation choisi, l'activation à distance de la prescription médicale informatisée, proposée dans le cadre de la présente invention, permet de garantir que l'application installée sur le terminal met en oeuvre les fonctionnalités logicielles en prenant en considération les données de prescription qui sont établies par le médecin.

Les différentes approches décrites ci-dessus et envisagées dans le cadre la présente invention se présentent comme autant de solutions fiables et sécurisées assurant aux professionnels de santé que le dispositif médical logiciel 400 fonctionne correctement en conformité avec les recommandations médicales du médecin.

Ces différentes approches permettent notamment d'établir de façon sécurisée le lien entre le patient et le médecin, de vérifier que la personne qui utilise le dispositif médical logiciel est bien le patient qui a consulté le médecin, de paramétrer à distance si nécessaire l'application logicielle prescrite en fonction de la pathologie à traiter, et de vérifier si celle-ci est conforme à la prescription médicale informatisée.

Ainsi, la présente invention par son approche innovante répond aux différentes problématiques sécuritaires et sanitaires soulevées aujourd'hui avec l'émergence des dispositifs médicaux logiciels.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Procédé d'activation d'une application logicielle (APP) apte à mettre en oeuvre sur un terminal de communication (300) des fonctionnalités logicielles (f1, f2, f3) pour accompagner un patient dans un traitement thérapeutique déterminé selon une prescription médicale informatisée (PMI),
ladite prescription (PMI), stockée dans une base de données (10) connectée à un serveur central distant (200), comprenant des données de prescription (D_PRESC) personnelles contenant des informations associées au traitement prescrit au patient, ledit procédé mis en oeuvre par des moyens informatiques comportant :
- suite à une installation (S2) de ladite application (APP) sur ledit terminal de communication (300), une étape d'authentification (S7, S8) auprès dudit serveur central (200) du patient via ledit terminal de communication (300) ;
- lorsque ledit patient s'est authentifié auprès dudit serveur central (200), une étape d'activation à distance (S11) de ladite application (APP) et desdites données de prescription (D_PRESC) sur le terminal de communication (300) pour autoriser la mise en oeuvre d'au moins une desdites fonctionnalités logicielles (f1, f2, f3) selon les données de prescription (D_PRESC) sur ledit terminal (300) ;
dans lequel l'étape d'activation (S11) comprend une transmission à destination du terminal de communication (300) d'un message d'activation (M_ACT) comprenant les données de prescription (D_PRESC) et un ordre d'activation (O_ACT).

2. Procédé selon la revendication 1, dans lequel l'étape d'authentification (S7, S8) comprend une comparaison (S7) par le serveur central (200) d'un code reçu en provenance du terminal de communication (300) avec un code de prescription unique (C_PRESC) enregistré dans ladite base de données (10).

3. Procédé selon la revendication 2, comprenant une étape de génération (S3) du code unique de prescription (C_PRESC) en fonction desdites données de prescription (D_PRESC) et d'un numéro unique d'identification (ID) associé au patient.

4. Procédé selon la revendication 2 ou 3, dans lequel le code unique de prescription (C_PRESC) se présente sous la forme d'un code matriciel à deux dimensions.

5. Procédé selon la revendication 4, dans lequel le code unique de prescription (C_PRESC) contient des informations relatives à des paramètres de configuration des fonctionnalités logicielles (f1, f2, f3).

6. Procédé selon la revendication 4 ou 5, comprenant une étape de lecture optique (S5) du code unique de prescription (C_PRESC) par le terminal de communication (300).

7. Procédé selon l'une quelconque des revendications précédentes, dans lequel les données de prescription (D_PRESC) comprennent des informations relatives :
- au patient,
- au praticien,
- aux caractéristiques techniques du terminal de communication servant de dispositif médical logiciel,
- aux paramètres de configuration des fonctionnalités logicielles (f1, f2, f3), et/ou
- au médicament prescrit par le praticien pour le traitement du patient comme par exemple le médicament en tant que tel, les règles d'auto-dosage dudit médicament par le patient lui-même, le dosage dudit médicament, les valeurs normales de mesure par le dispositif médical associé, et/ou la posologie associée audit médicament.

8. Procédé selon l'une quelconque des revendications 1 à 7, comprenant, suite à l'étape d'authentification (S7, S8), une étape d'émission (S9) au cours de laquelle le serveur central (200) émet un code d'accès à durée limitée (C_AUT) à destination dudit terminal de communication (300) pour autoriser l'activation de ladite application (APP) et desdites données de prescription (D_PRESC).

9. Programme d'ordinateur (PG) comportant des instructions adaptées pour l'exécution des étapes du procédé d'activation selon l'une quelconque des revendications 1 à 8 lorsque ledit programme d'ordinateur (PG) est exécuté par au moins un processeur.

10. Support d'enregistrement (CI) lisible par un ordinateur sur lequel est enregistré un programme d'ordinateur (PG) comprenant des instructions pour l'exécution des étapes du procédé d'activation selon l'une quelconque des revendications 1 à 8.

11. Système informatique d'activation (100) d'une application logicielle (APP) apte à mettre en oeuvre sur un terminal de communication (300) des fonctionnalités logicielles (f1, f2, f3) pour accompagner un patient dans un traitement thérapeutique déterminé selon une prescription médicale informatisée (PMI),
ladite prescription (PMI) comprenant des données de prescription personnelles (D_PRESC) contenant les informations associées au traitement prescrit au patient, ledit système (100) comprenant :
- une base de données (10) stockant ladite prescription médicale informatisée (PMI) ;
- des moyens d'authentification (40) aptes à coopérer avec des premiers moyens de communication (60) pour une authentification du patient via le terminal de communication (300) sur lequel est installée ladite application (APP) ;
- des moyens d'activation (50) aptes à coopérer avec lesdits premiers moyens de communication (60) pour activer à distance ladite application (APP) et lesdites données de prescription (D_PRESC) sur le terminal de communication (300) afin d'autoriser la mise en oeuvre desdites fonctionnalités (f1, f2, f3) selon les données de prescription (D_PRESC) sur ledit terminal (300) ;
dans lequel lesdits moyens d'activation (50) sont aptes à transmettre à destination du terminal de communication (300) un message d'activation (M_ACT) comprenant les données de prescription (D_PRESC) et un ordre d'activation (O_ACT).

12. Système (100) selon la revendication 11, comportant des moyens informatiques configurés pour la mise en oeuvre des étapes du procédé selon l'une quelconque des revendications 2 à 8.

13. Serveur informatique (200) comportant un système informatique d'activation (100) selon l'une des revendications 11 ou 12.

14. Dispositif médical (400) comportant un terminal de communication (300) sur lequel est installée une application logicielle (APP) apte à mettre en oeuvre des fonctionnalités logicielles (f1, f2, f3) pour accompagner un patient (P) dans un traitement thérapeutique déterminé selon une prescription médicale informatisée (PMI),
ladite prescription (PMI), stockée dans une base de données (10) connectée à un serveur central distant (200), comprenant des données de prescription (D_PRESC) personnelles contenant des informations associées au traitement prescrit au patient, ledit dispositif médical (400) comportant :
- un circuit électronique d'authentification (320) configuré pour autoriser une authentification auprès dudit serveur central (200) du patient via ledit terminal de communication (300) ; et
- un circuit électronique d'activation (330) configuré pour activer ladite application (APP) et lesdites données de prescription (D_PRESC) sur le terminal de communication (300) pour autoriser la mise en oeuvre d'au moins une desdites fonctionnalités logicielles (f1, f2, f3) selon les données de prescription (D_PRESC) sur ledit terminal (300) sur réception par le terminal de communication (300) d'un message d'activation (M_ACT) comprenant les données de prescription (D_PRESC) et un ordre d'activation (O_ACT).

15. Dispositif (400) selon la revendication 14, dans lequel le circuit électronique d'authentification (320) est apte à coopérer avec des deuxièmes moyens de communication (340) pour émettre à destination dudit serveur central (200) une requête (REQ) comprenant un code à comparer avec un code unique de prescription (C_PRESC) enregistré dans ladite base de données (10).

## Patentansprüche

1. Verfahren zum Aktivieren einer Software-Anwendung (APP), welche in der Lage ist, an einem Kommunikationsendgerät (300) Software-Funktionalitäten (f1, f2, f3) durchzuführen, um einen Patienten in einer therapeutischen Behandlung zu begleiten, welche gemäß einer computerisierten medizinischen Verschreibung (PMI) bestimmt wird,
wobei die Verschreibung (PMI), welche in einer Datenbank (10) gespeichert ist, welche mit einem entfernten zentralen Server (200) verbunden ist, persönliche Verschreibungsdaten (D_PRESC) umfasst, welche Informationen enthalten, welche der dem Patienten verschriebenen Behandlung zugeordnet sind, wobei das durch Computermittel durchgeführte Verfahren umfasst:
- einer Installation (S2) der Anwendung (APP) auf dem Kommunikationsendgerät (300) nachfolgend einen Authentifizierungsschritt (S7, S8) bei dem zentralen Server (200) des Patienten mittels des Kommunikationsendgeräts (300);
- wenn sich der Patient bei dem zentralen Server (200) authentifiziert hat, einen Schritt eines entfernten Aktivierens (S11) der Anwendung (APP) und der Verschreibungsdaten (D_PRESC) an dem Kommunikationsendgerät (300), um das Durchführen von wenigstens einer der Software-Funktionalitäten (f1, f2, f3) gemäß den Verschreibungsdaten (D_PRESC) an dem Endgerät (300) zu autorisieren;
wobei der Schritt des Aktivierens (S11) eine Übertragung mit dem Ziel des Kommunikationsendgeräts (300) einer Aktivierungsnachricht (M_ACT) umfasst, welche Verschreibungsdaten (D_PRESC) und einen Aktivierungsbefehl (O_ACT) umfasst.

2. Verfahren nach Anspruch 1, wobei der Authentifizierungsschritt (S7, S8) einen Vergleich (S7) durch den zentralen Server (200) eines Codes, welcher von dem Kommunikationsendgerät stammt, mit einem eindeutigen Verschreibungscode (C_PRESC) umfasst, welcher in der Datenbank (10) registriert ist.

3. Verfahren nach Anspruch 2, umfassend einen Schritt eines Erzeugens (S3) von eindeutigem Verschreibungscode (C_PRESC) als Funktion der Verschreibungsdaten (D_PRESC) und einer dem Patienten zugeordneten eindeutigen Identifikationszahl (ID).

4. Verfahren nach Anspruch 2 oder 3, wobei sich der eindeutige Verschreibungscode (C_PRESC) in der Form eines zweidimensionalen Matrixcodes darstellt.

5. Verfahren nach Anspruch 4, wobei der eindeutige Verschreibungscode (C_PRESC) Informationen bezüglich Konfigurationsparametern der Software-Funktionalitäten (f1, f2, f3) enthält.

6. Verfahren nach Anspruch 4 oder 5, umfassend einen Schritt eines optischen Lesens (S5) des eindeutigen Verschreibungscodes (C_PRESC) durch das Kommunikationsendgerät (300).

7. Verfahren nach einem der vorhergehenden Ansprüche, wobei die Verschreibungsdaten (D_PRESC) Informationen umfassen, bezüglich:
- des Patienten,
- des praktizierenden Arztes,
- der technischen Charakteristiken des Kommunikationsendgeräts, welches als medizinische Software-Vorrichtung dient,
- der Konfigurationsparameter der Software-Funktionalitäten (f1, f2, f3), und/oder
- des durch den praktizierenden Arzt für die Behandlung des Patienten verschriebenen Medikaments, wie beispielsweise das Medikament an sich, die Autodosierungs-Regeln des Medikaments für den Patienten selbst, die Dosierung des Medikaments, die normalen Messwerte für die zugeordnete medizinische Vorrichtung und/oder die dem Medikament zugeordnete Posologie.

8. Verfahren nach einem der Ansprüche 1 bis 7, umfassend auf den Authentifizierungsschritt (S7, S8) nachfolgend, einen Schritt eines Ausgebens (S9), während welchem der zentrale Server (200) einen Zugangscode mit begrenzter Dauer (C_AUT) an das Kommunikationsendgerät (300) gerichtet ausgibt, um die Aktivierung der Anwendung (APP) und der Verschreibungsdaten (D_PRESC) zu autorisieren.

9. Computerprogramm (PG), umfassend Anweisungen, welche dazu eingerichtet sind, die Schritte des Verfahrens zum Aktivieren nach einem der Ansprüche 1 bis 8 durchzuführen, wenn das Computerprogramm (PG) von wenigstens einem Prozessor ausgeführt wird.

10. Speichermedium (CI), welches durch einen Computer lesbar ist, auf welchem ein Computerprogramm (PG) registriert ist, welches Anweisungen zum Durchführen der Schritte des Verfahrens zum Aktivieren nach einem der Ansprüche 1 bis 8 umfasst.

11. Computer-Aktivierungssystem (100) für eine Software-Anwendung (APP), welche in der Lage ist, an einem Kommunikationsendgerät (300) Software-Funktionalitäten (f1, f2, f3) durchzuführen, um einen Patienten in einer therapeutischen Behandlung zu begleiten, welche gemäß einer computerisierten medizinischen Verschreibung (PMI) bestimmt wird,
wobei die Verschreibung (PMI) persönliche Verschreibungsdaten (D_PRESC) umfasst, welche Informationen enthalten, welche der dem Patienten verschriebenen Behandlung zugeordnet sind, wobei das System (100) umfasst:
- eine Datenbank (10), welche die computerisierte medizinische Verschreibung (PMI) speichert;
- Authentifizierungsmittel (40), welche in der Lage sind, mit ersten Kommunikationsmitteln (60) für eine Authentifizierung des Patienten über das Kommunikationsendgerät (300) zusammenzuwirken, auf welchem die Anwendung (APP) installiert ist;
- Aktivierungsmittel (50), welche in der Lage sind, mit den ersten Kommunikationsmitteln (60) zusammenzuwirken, um die Anwendung (APP) und die Verschreibungsdaten (D_PRESC) auf dem Kommunikationsendgerät (300) entfernt zu aktivieren, um das Durchführen der Funktionalitäten (f1, f2, f3) gemäß den Verschreibungsdaten (D_PRESC) an dem Endgerät (300) zu autorisieren;
wobei die Aktivierungsmittel (50) in der Lage sind, mit dem Ziel des Kommunikationsendgeräts (300) eine Aktivierungsnachricht (M_ACT) zu übertragen, welche Verschreibungsdaten (D_PRESC) und einen Aktivierungsbefehl (O_ACT) umfasst.

12. System (100) nach Anspruch 11, umfassend Computermittel, welche dazu eingerichtet sind, Schritte des Verfahrens nach einem der Ansprüche 2 bis 8 durchzuführen.

13. Computer-Server (200), umfassend ein Computer-Aktivierungssystem (100) nach einem der Ansprüche 11 oder 12.

14. Medizinische Vorrichtung (400), umfassend ein Kommunikationsendgerät (300), auf welchem eine Software-Anwendung (APP) installiert ist, welche in der Lage ist, Software-Funktionalitäten (f1, f2, f3) durchzuführen, um einen Patienten (P) in einer therapeutischen Behandlung zu begleiten, welche gemäß einer computerisierten medizinischen Verschreibung (PMI) bestimmt wird,
wobei die Verschreibung (PMI), welche in einer Datenbank (10) gespeichert ist, welche mit einem entfernten zentralen Server (200) verbunden ist, persönliche Verschreibungsdaten (D_PRESC) umfasst, welche Informationen enthalten, welche der dem Patienten verschriebenen Behandlung zugeordnet sind, wobei die medizinische Vorrichtung (400) umfasst:
- eine elektronische Authentifizierungsschaltung (320), welche dazu eingerichtet ist, eine Authentifizierung bei dem zentralen Server (200) des Patienten mittels des Kommunikationsendgeräts (300) zu autorisieren; und
- eine elektronische Aktivierungsschaltung (330), welche dazu eingerichtet ist, die Anwendung (APP) und die Verschreibungsdaten (D_PRESC) auf dem Kommunikationsendgerät (300) zu aktivieren, um das Durchführen der Software-Funktionalitäten (f1, f2, f3) gemäß den Verschreibungsdaten (D_PRESC) an dem Endgerät (300) auf den Empfang einer Aktivierungsnachricht (M_ACT), welche Verschreibungsdaten (D_PRESC) und einen Aktivierungsbefehl (O_ACT) umfasst, an dem Kommunikationsendgerät (300) hin zu autorisieren.

15. Vorrichtung (400) nach Anspruch 14, wobei die elektronische Authentifizierungsschaltung (320) in der Lage ist, mit den zweiten Kommunikationsmitteln (340) zusammenzuwirken, um mit dem Ziel des zentralen Servers (200) eine Anfrage (REQ) auszugeben, welche einen Code zum Vergleichen mit einem eindeutigen Verschreibungscode (C_PRESC) umfasst, welcher in der Datenbank (10) registriert ist.

## Claims

1. Method for activating a software application (APP) capable of executing software functions (f1, f2, f3) on a communication terminal (300) in order to assist a patient in a therapeutic treatment determined according to a computerised medical prescription (PMI),
said prescription (PMI), stored in a database (10) connected to a remote central server (200), comprising personal prescription data (D_PRESC) containing information associated with the treatment prescribed to the patient,
with said method implemented by computer means comprising:
- following an installation (S2) of said application (APP) on said communication terminal (300), a step of authentication (S7, S8) with said central server (200) of the patient via said communication terminal (300);
- when said patient has authenticated himself with said central server (200), a step of remotely activating (S11) said application (APP) and said prescription data (D_PRESC) on the communication terminal (300) in order to authorise the execution of at least one of said software functions (f1, f2, f3) according to the prescription data (D_PRESC) on said terminal (300);
wherein the step of activating (S11) comprises a transmission intended for the communication terminal (300) of an activation message (M_ACT) that comprises the prescription data (D_PRESC) and an activation order (O_ACT).

2. Method according to claim 1, wherein the step of authenticating (S7, S8) comprises a comparison (S7) by the central server (200) of a code received coming from the communication terminal (300) with a unique prescription code (C_PRESC) recorded in said database (10).

3. Method according to claim 2, comprising a step of generating (S3) the unique prescription code (C_PRESC) according to said prescription data (D_PRESC) and a unique identification number (ID) associated with the patient.

4. Method according to claim 2 or 3, wherein the unique prescription code (C_PRESC) has the form of a two-dimensional matrix code.

5. Method according to claim 4, wherein the unique prescription code (C_PRESC) contains information concerning configuration settings of the software functions (f1, f2, f3).

6. Method according to claim 4 or 5, comprising a step of optical reading (S5) of the unique prescription code (C_PRESC) by the communication terminal (300).

7. Method as claimed in any preceding claim, wherein the prescription data (D_PRESC) includes information concerning:
- the patient,
- the practitioner,
- the technical characteristics of the communication terminal used as a medical software device,
- the configuration settings of the software functions (f1, f2, f3), and/or
- medication prescribed by the practitioner for the treatment of the patient such as for example the medication as such, the self-dosage rules for said medication by the patient himself, the dosage of said medication, the normal measurement values by the associated medical device, and/or the posology associated with said medication.

8. Method according to any of claims 1 to 7, comprising, following the step of authentication (S7, S8), a step of emitting (S9) during which the central server (200) emits an access code with a limited duration (C_AUT) to said communication terminal (300) in order to authorise the activation of said application (APP) and of said prescription data (D_PRESC).

9. Computer program (PG) comprising instructions suitable for the execution of the steps of the method for activation according to any of claims 1 to 8 when said computer program (PG) is executed by at least one processor.

10. Recording support (CI) that can be read by a computer whereon a computer program (PG) is recorded comprising instructions for the execution of the steps of the method of activation according to any of claims 1 to 8.

11. Computer system for activation (100) of a software application (APP) capable of executing software functions (f1, f2, f3) on a communication terminal (300) in order to assist a patient in a therapeutic treatment determined according to a computerised medical prescription (PMI),
said prescription (PMI) comprising personal prescription data (D_PRESC) containing information associated with the treatment prescribed to the patient,
said system (100) comprising:
- a database (10) that stores said computerised medical prescription (PMI);
- means for authentication (40) able to cooperate with first means of communication (60) for an authentication of the patient via the communication terminal (300) whereon said application (APP) is installed;
- activation means (50) able to cooperate with said first means of communication (60) in order to remotely activate said application (APP) and said prescription data (D_PRESC) on the communication terminal (300) in order to authorise the execution of said functions (f1, f2, f3) according to the prescription data (D_PRESC) on said terminal (300);
wherein said activation means (50) are able to transmit to the communication terminal (300) an activation message (M_ACT) that comprises the prescription data (D_PRESC) and an activation order (O_ACT).

12. System (100) according to claim 11, comprising computer means configured for the implementation of the steps of the method according to any of claims 2 to 8.

13. Computer server (200) comprising a computer system (100) for activation according to one of claims 12 or 11.

14. Medical device (400) comprising a communication terminal (300) whereon a software application (APP) is installed that can execute software functions (f1, f2, f3) in order to assist a patient (P) in a therapeutic treatment determined according to a computerised medical prescription (PMI),
said prescription (PMI), stored in a database (10) connected to a remote central server (200), comprising personal prescription data (D_PRESC) containing information associated with the treatment prescribed to the patient,
said medical device (400) comprising:
- an electronic authentication circuit (320) configured to authorise an authentication with said central server (200) of the patient via said communication terminal (300); and
- an electronic activation circuit (330) configured to activate said application (APP) and said prescription data (D_PRESC) on the communication terminal (300) in order to authorise the execution of at least one of said software functions (f1, f2, f3) according to the prescription data (D_PRESC) on said terminal (300) on reception by the communication terminal (300) of an activation message (M_ACT) that comprises the prescription data (D_PRESC) and an activation order (O_ACT).

15. Device (400) according to claim 14, wherein the electronic authentication circuit (320) is able to cooperate with second means of communication (340) in order to emit to said central server (200) a request (REQ) comprising a code to be compared with a unique prescription code (C_PRESC) recorded in said database (10).
